# EUROPÄISCHE PATENTSCHRIFT

(11) **EP 1 368 008 B1**
(45) Veröffentlichungstag und Bekanntmachung des Hinweises auf die Patenterteilung: **27.09.2006**
(21) Anmeldenummer: 02719920.7
(22) Anmeldetag: 26.02.2002
(51) Int. Cl.: A61K 9/70, A61K 31/4704, A61P 25/18

(54) **TRANSDERMALES THERAPEUTISCHES SYSTEM ZUR VERABREICHUNG DES PARTIELLEN DOPAMIN-D2-AGONISTEN ARIPIPRAZOL**
TRANSDERMAL THERAPEUTIC SYSTEM FOR THE ADMINISTRATION OF THE PARTIAL DOPAMINE-D2 AGONIST ARIPIPRAZOL
SYSTEME THERAPEUTIQUE TRANSDERMIQUE POUR L'ADMINISTRATION D'AGONISTE PARTIEL DE LA DOPAMINE D2 ARIPIPRAZOL

(30) Priorität: 07.03.2001 DE 10110953
(43) Veröffentlichungstag der Anmeldung: 10.12.2003
(73) Patentinhaber: LTS LOHMANN Therapie-Systeme AG, 56626 Andernach (DE)
(72) Erfinder: SELZER, Thorsten, 60388 Frankfurt (DE)
(74) Vertreter: Flaccus, Rolf-Dieter
(86) Internationale Anmeldenummer: PCT/EP2002/002002
(87) Internationale Veröffentlichungsnummer: WO 2002/069941

(56) Entgegenhaltungen:
- US-A- 5 807 570
- MALLIKAARJUM S ET AL: "Pharmacokinetics, tolerability, and safety of aripiprazole following single and multiple oral dose administration." EUROPEAN NEUROPSYCHOPHARMACOLOGY, Bd. 10, Nr. Supplement 3, September 2000 (2000-09), Seiten S306-S307, XP002205415 13th Congress of the European College of Neuropsychopharmacology;Munich, Germany; September 09-13, 2000 ISSN: 0924-977X
- DAAS DEN I ET AL: "TRANSDERMAL ADMINISTRATION OF THE DOPAMINE AGONIST N-0437 AND SEVEN ESTER PRODRUGS: COMPARISON WITH ORAL ADMINISTRATION IN THE 6-OHDA TURNING MODEL" NAUNYN-SCHMIEDEBERG'S ARCHIVES OF PHARMACOLOGY, SPRINGER, BERLIN, DE, Bd. 342, Nr. 6, Dezember 1990 (1990-12), Seiten 655-659, XP001062117 ISSN: 0028-1298
- LAWLER, CINDY P. (1) ET AL: "Interactions of the novel antipsychotic aripiprazole (OPC-14597) with dopamine and serotonin receptor subtypes." NEUROPSYCHOPHARMACOLOGY, (JUNE, 1999) VOL. 20, NO. 6, PP. 612-627. , XP002205416

## Beschreibung

Die vorliegende Erfindung betrifft transdermale therapeutische Systeme (TTS) zur Verabreichung von Wirkstoffen aus der Gruppe der partiellen Dopamin-D2-Agonisten insbesondere von Aripiprazol an die Haut von Patienten. Sie betrifft ferner die Verwendung solcher TTS zur medikamentösen Behandlung von Patienten, die an schizophrenen Psychosen erkrankt sind.

Nach dem heute gültigen Denkmodell werden schizophrene Psychosen als klinische Manifestation eines Ungleichgewichts im komplizierten Netzwerk der Neurotransmitter in den verschiedenen Hirnregionen angesehen. Dem Neurotransmitter Dopamin kommt dabei eine zentrale Rolle als Modulator im Zusammenspiel zu.
Klassische Neuroleptika bewirken eine nahezu vollständige, unspezifische Blockade der Dopamin-D2-Rezeptoren. Ihre gute Wirksamkeit gegenüber der produktiven Positivsymptomatik ist zwar erwiesen, allerdings erzeugen diese Wirkstoffe gleichzeitig inakzeptable extrapyramidal-motorische Nebenwirkungen.
Deshalb besteht das bevorzugte Ziel darin, eine Normalisierung - statt einer Blockade - der neuronalen Erregungsleitung herbeizuführen. Die Dopamin-Aktivität soll in denjenigen Hirnarealen vermindert werden, wo sie zu stark ist, aber nicht dort, wo sie normal ist. Diese Forderung wird durch die Wirkstoffgruppe der partiellen D2-Agonisten weitgehend erfüllt.

Partielle Dopamin-D2-Agonisten, zu denen beispielsweise der zu der allgemeinen Stoffklasse der Phenylpiperazinylchinolinone gehörende Wirkstoff Aripiprazol zählt, zeichnen sich dadurch aus, daß sie die postsynaptischen Dopamin-D2-Rezep-toren blockieren und gleichzeitig die präsynaptischen Autorezeptoren stimulieren. Auf diese Weise wird die übersteigerte Dopamin-Aktivität unterdrückt und gleichzeitig das Risiko extrapyramidal-motorischer Nebenwirkungen vermindert.
Die therapeutische Wirksamkeit und Verträglichkeit von Aripiprazol wurde in den USA in einer randomisierten multizentrischen Doppelblind-Studie im Vergleich zu Haloperidol und einem Plazebo untersucht und bestätigt.

Allerdings weist insbesondere der partielle Dopamin-D2-Agonist Aripiprazol einige Nachteile auf, die sich aus der Pharmakokinetik ergeben. Aripiprazol unterliegt bei oraler Gabe einer ausgeprägten Metabolisierung während der ersten Darm-Leber-Passage (First-Pass-Effekt) und besitzt eine geringe Plasma-Halbwertszeit. Um einen therapeutisch wirksamen Plasmaspiegel aufrechtzuerhalten, ist deshalb eine relativ hohe Applikationsfrequenz bei oraler Verabreichung erforderlich.

MALLIKAARJUM S ET AL: "Pharmacokinetics, tolerability, and safety of aripiprazole following single and multiple oral dose administration." EUROPEAN NEUROPSYCHOPHARMACOLOGY, Bd. 10, Nr. Supplement 3, September 2000 (2000-09), Seiten S306-S307, 13th Congress of the European College of Neuropsychopharmacology;Munich, Germany; September 09-13, 2000 ISSN: 0924-977X offenbart die Ergebnisse einer oralen Verabreichung von Aripiprazol im Rahmen einer klinischen Multizenter Studie bei schizophrenen Patienten.

Die der Erfindung zugrunde liegende Aufgabe bestand deshalb darin, den Wirkstoff Aripiprazol (7-(4-(4-(2,3-Dichlorophenyl)-1-piperazinyl)butoxy)-3,4-dihydro-2(1H)-chinolinon oder einen anderen Wirkstoff aus der Gruppe der partiellen D2-Agonisten in einer Darreichungsform bereitzustellen, durch welche die vorstehend genannten Nachteile vermieden werden können, und die sich in vorteilhafter Weise für die therapeutische Behandlung von schizophrenen Psychosen eignet.

Diese Aufgabe wird gelöst durch transdermale therapeutische Systeme (TTS) in Pflasterform nach den Ansprüchen 1 bis 13. Diese ermöglichen einen für therapeutische Zwecke ausreichenden Wirkstoff-Flux in vivo und können mittels gängiger Herstellungsverfahren produziert werden.

Die erfindungsgemäßen, Aripiprazol enthaltenden TTS geben nach der Applikation den enthaltenen Wirkstoff an die Haut des Patienten ab, so daß der Wirkstoff systemisch verfügbar wird. Durch die direkte Abgabe des Wirkstoffs in das Blutgefäßsystem wird die bei oraler Verabreichung auftretende schnelle Metabolisierung infolge des First-Pass-Effekts vermieden. Zudem wird durch die erfindungsgemäßen TTS eine konstante Abgabe des/der enthaltenen partiellen D2-Agonisten während des Applikationszeitraums gewährleistet. Auf diese Weise läßt sich - bei niedriger Applikationsfrequenz - die Aufrechterhaltung eines relativ gleichbleibenden Plasmaspiegels erreichen. Die rasche Elimination des partiellen Dopamin-D2-Agonisten (z. B. Aripiprazol) wird durch ständiges Nachliefern des Wirkstoffs aus dem Wirkstoffreservoir des TTS kompensiert.
Durch die transdermale Verabreichung werden außerdem Probleme wie z. B. gastrointestinale Intoleranz, niedrige enterale Absorption oder geringe perorale Verfügbarkeit umgangen.

Gegenstand der vorliegenden Erfindung ist somit ein transdermales therapeutisches System (TTS) in Pflasterform, welches die im Oberbegriff des Anspruchs 1 genannten Merkmale eines TTS aufweist, und dessen Wirkstoffreservoir mindestens einen Wirkstoff aus der Gruppe der partiellen Dopamin-D2-Agonisten enthält wobei mindestens einer der partiellen Dopamin D2-Agonisten Aripiprazol ist. Weitere bevorzugte Ausführungsformen der Erfindung sind in den Unteransprüchen beschrieben.

Der Aufbau der erfindungsgemäßen TTS umfaßt eine wirkstoffundurchlässige Rückschicht, ein Wirkstoffreservoir und eine ablösbare Schutzschicht. Das wirkstoffhaltige Reservoir kann selbst haftklebende Eigenschaften haben, oder es ist eine Haftklebeschicht vorgesehen, welche die Befestigung des TTS auf der Haut ermöglicht. Die mit dem Wirkstoffreservoir verbundene Rückschicht bedeckt das TTS auf der hautabgewandten Seite. Die ablösbare und arzneistoffundurchlässige Schutzschicht bedeckt während der Lagerung die hautseitige, haftklebende Oberfläche des TTS und wird vor der Applikation abgelöst.
Die Erfindung umfaßt sowohl TTS, die als Matrixsysteme ausgebildet sind, als auch solche, die als Membransysteme ausgebildet sind.

Die erfindungsgemäßen TTS können sowohl in Form von Matrixsystemen als auch in Form von Beutel- oder Membransystemen eingesetzt werden. Im Falle eines Matrixsystems kann die Wirkstoffmatrix beispielsweise eine Kunststoff- oder Kunstharzmatrix sein, welche als Wirkstoffreservoir dient und den Wirkstoff in gelöster oder dispergierter Form enthält. Sie ist vorzugsweise haftklebend und kann sowohl ein- als auch zwei- oder mehrschichtig ausgebildet sein. Der Begriff "Matrixsysteme" schließt auch solche Ausführungsformen mit ein, bei welchen das Wirkstoffreservoir ein Fasermaterial, beispielsweise ein Baumwollgewebe oder -vlies enthält, an welches der Wirkstoff, z. B. Aripiprazol, adsorbiert ist. Dieses Fasermaterial kann in einer Kunststoff- oder Kunstharzmatrix eingebettet sein.

Grundsätzlich kommen für die Herstellung der wirkstoffhaltigen Matrix oder Matrixschichten eine Vielzahl von Polymeren, Harzen und Zusatzstoffen in Betracht, die dem Fachmann bekannt sind, wobei allerdings darauf zu achten ist, daß diese Stoffe - sofern sie mit der Haut in Berührung kommen können - hautverträglich sind, und daß die Formulierung geeignet ist, den Wirkstoff Aripiprazol oder einen anderen Dopamin-D2-Agonisten an die Haut abzugeben.

Geeignete Grundpolymere für die Herstellung der Wirkstoffmatrix oder der haftklebenden Schicht der erfindungsgemäßen TTS sind Polymere auf der Basis von Acrylsäure und deren Estern, Isobutylen, Ethylen-Vinylacetat-Copolymere, natürliche Kautschuke, synthetische Kautschuke wie Styrol-Dien-Copolymere, insbesondere Styrol-Butadien-Blockcopolymere, Isopren-Blockpolymere, Acrylnitril-Butadien-Kautschuk, Butylkautschuk oder Neopren-Kautschuk, sowie Haftkleber auf Silikonbasis und Heißschmelzkleber. Unter den Begriff "Heißschmelzkleber" fallen alle Kleber, die nicht durch Lösemittel, sondern durch Schmelzen bei erhöhten Temperaturen, vorzugsweise im Bereich von 60-200 °C, verflüssigt werden. Als Heißschmelzkleber eignen sich insbesondere Mischungen aus Estern des hydrierten Kolophoniums mit Cellulosederivaten. Die genannten Grundpolymere können auch in Form geeigneter Mischungen verwendet werden.

Neben den genannten Polymeren können auch weitere, dem Fachmann bekannte Polymere als Grundpolymere für die Herstellung der Matrix oder der Haftklebeschicht verwendet werden, vorausgesetzt, diese sind mit dem Wirkstoff Aripiprazol bzw. dem jeweils verwendeten partiellen Dopamin-D2-Agonisten kompatibel.

Der Wirkstoff Aripiprazol, und gegebenenfalls ein anderer partieller Dopamin-D2-Agonist, ist im einfachsten Fall in einer Lösung oder Schmelze von Grundpolymeren grob, kolloidal oder molekular dispergiert. Die weitere Herstellung des TTS kann auf die Weise erfolgen, daß diese wirkstoffhaltige Mischung auf eine geeignete Unterlage, beispielsweise auf eine mit einer Silikonschicht versehenen thermoplastische Folie, beschichtet und - gegebenenfalls nach Abdampfen der Lösemittelbestandteile - mit einer weiteren Folie abgedeckt wird, welche die spätere Rückseite des TTS darstellt. Durch Stanzen flächiger Gebilde in der gewünschten geometrischen Form werden TTS aus einem solchen Laminat hergestellt. Die als Hilfsstoffe, wie Weichmacher, Klebrigmacher, Lösungsvermittler, Stabilisatoren, Füllstoffe, Trägerstoffe und Permeationsbeschleuniger, in Frage kommenden pharmazeutisch unbedenklichen Substanzen sind dem Fachmann grundsätzlich bekannt.

Gemäß einer weiteren Ausführungsform ist vorgesehen, daß der Wirkstoff in einem beutelförmigen Reservoir des erfindungsgemäßen TTS vorliegt. Dieses ist mit einer fließfähigen, z. B. viskosen oder hochviskosen oder halbfesten Kunststoffmatrix oder einer Lösung davon gefüllt, die den Wirkstoff enthält. Besonders vorteilhaft ist es, wenn das Wirkstoffreservoir einen Gelbildner enthält. Die der Haut abgewandte Beutelrückseite muß dabei wirkstoffundurchlässig, die der Haut zugewandte Seite muß wirkstoffdurchlässig sein. Zur Steuerung der Wirkstofffreisetzung kann auf der der Haut zugewandten Seite des Beutels eine wirkstoffdurchlässige Membran angebracht sein ("Membransystem"). Geeignete Materialien für die Herstellung des Beutels und für die Membran sowie Gelbildner sind dem Fachmann bekannt.

Eine bevorzugte Ausführungsform der Erfindung besteht darin, daß der Wirkstoff Aripiprazol, oder ein anderer Wirkstoff aus der Gruppe der partiellen Dopamin-D2-Agonisten, im Reservoir des TTS im gelösten Zustand vorliegt, wobei die Formulierung möglichst einen Lösungsvermittler enthalten sollte. Bevorzugte Beispiele für Lösungsvermittler sind mehrwertige Alkohole, insbesondere 1,2-Propandiol, Butandiole, Glycerin, Polyethylenglykol 400, Tetrahydrofurfurylalkohol, Diethylenglykolmonoether, Diethyltoluamid und Monoisopropyliden-glycerin; 1,2-Propandiol wird besonders bevorzugt eingesetzt. Als vorteilhaft hat sich herausgestellt, wenn der Anteil des Lösungsvermittlers 1 bis 50 Gew.-% beträgt, besonders bevorzugt 5 bis 35 Gew.-%, bezogen auf das Gesamtgewicht des Wirkstoffreservoirs. Zu berücksichtigen ist, daß einige der genannten Lösungsvermittler, wie z. B. 1,2-Propandiol, zugleich als permeationsfördernde Stoffe wirken können.

Um einen hohen Wirkstoff-Flux durch die Haut zu erzielen, hat es sich insbesondere bei Matrixsystemen als vorteilhaft erwiesen, wenn der wirkstoffhaltigen Matrix permeationsfördernde Stoffe in einer Menge von 0,1 bis 25 Gew.-% , vorzugsweise von 1 bis 10 Gew.-% zugesetzt werden, jeweils bezogen auf das Gesamtgewicht des Wirkstoffmatrix. Bevorzugte Beispiele für hautpermeationsfördernde Zusatzstoffe sind Fettalkohole wie Decanol und Dodecanol, sowie Fettsäuren, wie z. B. Ölsäure oder Myristinsäure, sowie Polyoxyethylenfettalkoholether, vorzugsweise Polyoxylaurylether (z. B.
Brij® ), sowie Polyoxyethylenfettsäureester, Fettsäureester von Sorbitanmonolaurat, Ester von langkettigen Fettsäuren mit Methyl-, Ethyl- oder Isopropylalkohol, Ester von Fettalkoholen mit Essigsäure oder Milchsäure, sowie Ölsäurediethanolamin. Die genannten permeationsfördernden Stoffe können entweder einzeln oder als Mischung zugesetzt werden.

Um eine hohe Wirkstoff-Freisetzungsrate zu erzielen, wird eine möglichst hohe Wirkstoffkonzentration in der Wirkstoffmatrix bzw. den wirkstoffhaltigen Schichten bevorzugt. Dabei ist allerdings zu beachten, daß bei zu hohen Konzentrationen die physikalische Stabilität des Wirkstoffs beeinträchtigt werden kann. Bei den erfindungsgemäßen TTS werden deshalb Wirkstoffkonzentrationen im Bereich von 0,1 bis 50 Gew.-%, insbesondere von 1 bis 10 Gew.-% angewandt, jeweils bezogen auf die Gesamtmasse des Wirkstoffreservoirs.
Nach der Erfindung ist ferner vorgesehen, daß der Wirkstoff Aripiprazol, oder ein anderer partieller Dopamin-D2-Agonist, in Kombination mit mindestens einem weiteren Wirkstoff vorliegt. Dabei handelt es sich bevorzugt um einen Wirkstoff oder Wirkstoffe aus der Stoffklasse der Phenothiazine und/oder deren Analoga, und/oder aus der Klasse der Butyrophenone und/oder aus der Klasse der Diphenylbutylpiperidine.

Ferner kann die Wirkstoffmatrix oder einzelne Schichten der Matrix Weichmacher enthalten, welche dem Fachmann grundsätzlich bekannt sind. Die Konzentration dieser Weichmacher kann bis zu 30 Gew.-% betragen und liegt vorzugsweise zwischen 5 und 20 Gew.-%, jeweils bezogen auf die Wirkstoffmatrix. Die Weichmacher können beispielsweise aus den Gruppen der Kohlenwasserstoffe, Alkohole, Carbonsäuren und ihre Derivate, Ether, Ester oder Amine ausgewählt sein.

Um eine Steuerung der Wirkstoff-Freisetzung zu ermöglichen, sofern dies nicht durch andere Mechanismen bewirkt wird, kann das Wirkstoffreservoir an der hautnahen, Wirkstoff abgebenden Seite auch mit einer Steuermembran versehen werden, welche die Abgabe des Wirkstoffs an die Haut steuert ("Membransystem").

Die Erfindung schließt auch solche Ausführungsformen mit ein, bei denen die Wirkstoffmatrix einen zwei- oder mehrschichtigen Aufbau aufweist. Beispielsweise können die verschiedenen Matrixschichten Polymerbestandteile aus der Gruppe der substituierten Cellulosen enthalten, vorzugsweise der Methyl- und Ethylcellulosen.
Die einzelnen Matrixschichten können dabei so gestaltet sein, daß sie sich hinsichtlich ihrer Polymer- oder Haftkleberzusammensetzung, der Wirkstoffkonzentration, der Konzentration an permeationsfördernden Zusatzstoffen oder an Lösungsvermittlern unterscheiden. Je nach dem beabsichtigten Anwendungszweck lassen sich die genannten Konzentrationen in den einzelnen Matrixschichten in der Weise unterschiedlich gestalten, daß die Konzentrationen von der hautfernen Schicht in Richtung zur hautnahen Matrixschicht kleiner oder größer werden, je nachdem, ob eine besondere Langzeitwirkung oder eine Initialwirkung angestrebt wird.

Die Befestigung der erfindungsgemäßen TTS auf der Haut kann auf unterschiedliche Weise erreicht werden. Beispielsweise besteht die Möglichkeit, daß die Wirkstoffmatrix selbst aus einem Haftkleber oder aus einer Mischung von haftklebenden Polymeren besteht. Auch die Befestigung des TTS auf der Haut mittels einer zusätzlichen, wirkstofffreien Haftkleberschicht ist möglich. Darüber hinaus ist es auch denkbar, das bei solchen erfindungsgemäßen TTS, die mit einer Steuermembran ausgestattet sind, die Befestigung auf der Haut mittels eines Klebstoffrands bewirkt wird. Dieser Klebstoffrand berührt die wirkstoffabgebende Fläche nicht, steht also nicht mit der Steuermembran in Verbindung.

Die erfindungsgemäßen TTS weisen neben dem Wirkstoffreservoir außerdem eine wirkstoffundurchlässige Rückschicht sowie eine ebenfalls wirkstoffundurchlässige ablösbare Schutzschicht oder Abziehfolie auf.
Als Materialien für die Rückschicht eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Ethylen-Vinylacetat-Copolymere, Polyvinylacetat, Polyethylen, Polypropylen, Polyurethane, Cellulosederivate und viele andere mehr. Im Einzelfall kann die Rückschicht mit einer zusätzlichen Auflage versehen werden, z. B. durch Bedampfung mit Metallen, insbesondere Aluminium.

Für die ablösbare Schutzschicht können grundsätzlich dieselben Materialien verwendet werden wie für die Rückschicht, vorausgesetzt, daß sie durch eine geeignete Oberflächenbehandlung, wie z. B. Silikonisierung, ablösbar ausgerüstet ist. Es können aber auch andere ablösbare Schutzschichten wie z. B. mit Polytetrafluorethylen behandeltes Papier oder Cellophan® (Cellulosehydrat) verwendet werden.

Die erfindungsgemäßen TTS eignen sich in vorteilhafter Weise zur Akut- und Langzeittherapie bei schizophrenen Psychosen. Hierbei wird das den Wirkstoff Aripiprazol und gegebenenfalls einen anderen partiellen Dopamin-D2-Agonisten enthaltende TTS auf die Haut eines solchen Patienten appliziert und dort für einen Zeitraum von mindestens 8 Stunden belassen. Die Applikationsdauer kann bis zu drei Tage betragen.

Die erfindungsgemäßen TTS können beispielsweise wie folgt hergestellt werden:

### Beispiel

50 g des Wirkstoffs Aripiprazol sowie 20 g eines geeigneten permeationsfördernden Stoffes (z. B. Brij® 30) werden in 200 g 1,2-Propandiol gelöst. Diese Lösung wird mittels einer geeigneten Rührapparatur in einen Silikonkleber (Nr. 4301, Fa. Dow Corning, USA) als Grundpolymer gegeben und darin dispergiert, so daß eine möglichst homogene Flüssig-Flüssig-Dispersion entsteht. Diese Dispersion wird mit einer geeigneten Vorrichtung gleichmäßig auf eine Trägerfolie (z. B. aus Polyethylenterephthalat) beschichtet. Anschlie-ßend wird durch kontrollierte Trocknung das Lösungsmittel des Silikonklebers sowie etwaige Anteile des Propandiols entfernt. Das so erhaltene Laminat wird anschließend mit einer weiteren Folie aus Polyethylenterephthalat zukaschiert. Zuletzt werden TTS einer bestimmten Fläche ausgestanzt und in ein geeignetes Packmittel verpackt.

## Patentansprüche

1. Transdermales therapeutisches System (TTS) zur Verabreichung mindestens eines partiellen Dopamin-D2-Agonisten, welches eine wirkstoffundurchlässige Rückschicht, ein Wirkstoffreservoir und eine ablösbare Schutzschicht aufweist, wobei das Wirkstoffreservoir haftklebend ist oder das TTS mindestens eine haftklebende Schicht aufweist, und wobei das Wirkstoffreservoir als Matrixsystem oder als Membransystem ausgebildet ist, **dadurch gekennzeichnet, daß** mindestens einer der im Wirkstoffreservoir enthaltenen partiellen Dopamin-D2-Agonisten Aripiprazol ist.

2. TTS nach Anspruch 1, **dadurch gekennzeichnet, daß** das Wirkstoffreservoir als ein-, zwei- oder mehrschichtige Wirkstoffmatrix ausgebildet ist.

3. TTS nach Anspruch 2, **dadurch gekennzeichnet, daß** die Wirkstoffmatrix eine Kunststoff- oder Kunstharzmatrix ist, vorzugsweise eine haftklebende Matrix, wobei das/die Grundpolymer(e) dieser Matrix bevorzugt aus der Gruppe ausgewählt ist/sind, die Polymere auf der Basis von Acrylsäure und deren Estern, Isobutylen, Ethylen-Vinylacetat-Copolymere, natürliche Kautschuke, synthetische Kautschuke wie Styrol-Dien-Copolymere, insbesondere Styrol-Butadien-Blockcopolymere, Isopren-Blockpolymere, Acrylnitril-Butadien-Kautschuk, Butylkautschuk oder Neopren-Kautschuk, sowie Haftkleber auf Silikonbasis sowie Heißschmelzkleber, vorzugsweise Mischungen aus Estern des hydrierten Kolophoniums mit Cellulosederivaten, umfaßt.

4. TTS nach einem der vorangehenden Ansprüche, **dadurch gekennzeichnet, daß** das Wirkstoffreservoir ein Fasermaterial, Gewebe oder Vlies enthält, an welches der Wirkstoff adsorbiert ist.

5. TTS nach Anspruch 1, **dadurch gekennzeichnet, daß** das Wirkstoffreservoir als beutelförmiges Reservoir ausgebildet ist, welches den Wirkstoff in einer fließfähigen, viskosen, halbfesten, gel-artigen oder flüssigen Zubereitung oder Lösung enthält und das hautseitig von einer wirkstoffdurchlässigen Schicht, auf der hautabgewandten Seite von einer wirkstoffundurchlässigen Schicht begrenzt ist.

6. TTS nach einem vorangehenden Ansprüche, **dadurch gekennzeichnet, daß** es zusätzlich eine wirkstoffdurchlässige Membran aufweist, welche die Geschwindigkeit der Wirkstoffabgabe modifiziert oder steuert.

7. TTS nach einem der vorangehenden Ansprüche, **dadurch gekennzeichnet, daß** Aripiprazol in einer Konzentration im Bereich von 0,1 bis 50 Gew.-%, bevorzugt von 1 bis 10 Gew.-% enthalten ist, jeweils bezogen auf die Gesamtmasse des Wirkstoffreservoirs.

8. TTS nach einem der vorangehenden Ansprüche, **dadurch gekennzeichnet, daß** Aripiprazol im gelösten Zustand im Wirkstoffreservoir vorliegt.

9. TTS nach einem der vorangehenden Ansprüche, **dadurch gekennzeichnet, daß** das Wirkstoffreservoir mindestens einen Lösungsvermittler enthält, vorzugsweise in einer Menge von 1 bis 50 Gew.-%, besonders bevorzugt von 5 bis 35 Gew.-%, jeweils bezogen auf das Gesamtgewicht des Wirkstoffreservoirs.

10. TTS nach Anspruch 9, **dadurch gekennzeichnet, daß** der/die Lösungsvermittler aus der Gruppe ausgewählt ist/sind, die mehrwertige Alkohole, vorzugsweise 1,2-Propandiol, Butandiole, Glycerin, Polyethylenglykol 400, Tetrahydrofurfurylalkohol, Diethylenglykolmonoether, Diethyltoluamid und Monoisopropyliden-glycerin umfaßt.

11. TTS nach einem der vorangehenden Ansprüche, **dadurch gekennzeichnet, daß** das Wirkstoffreservoir mindestens einen permeationsfördernden Stoff enthält, vorzugsweise in einer Menge von 0,1 bis 25 Gew.-%, besonders bevorzugt von 1 bis 10 Gew.-%, jeweils bezogen auf das Gesamtgewicht des Wirkstoffreservoirs.

12. TTS nach Anspruch 8, **dadurch gekennzeichnet, daß** der/die permeationsfördernde(n) Stoff(e) aus der Gruppe ausgewählt ist/sind, die Fettalkohole, vorzugsweise Decanol und Dodecanol, sowie Fettsäuren, vorzugsweise ölsäure, Myristinsäure, sowie Polyoxyethylenfettalkoholether, vorzugsweise Polyoxylaurylether, sowie Polyoxyethylenfettsäureester, Fettsäureester von Sorbitanmonolaurat, Ester von langkettigen Fettsäuren mit Methyl-, Ethyl- oder Isopropylalkohol, Ester von Fettalkoholen mit Essigsäure oder Milchsäure, sowie Ölsäurediethanolamin umfaßt.

13. TTS nach einem der vorangehenden Ansprüche, **dadurch gekennzeichnet, daß** es zusätzlich mindestens einen Wirkstoff enthält, der aus der Gruppe ausgewählt ist, die Phenothiazine und deren Analoga, Butyrophenone und Diphenylbutylpiperidine umfaßt.

14. Verwendung von Aripiprazol zur Herstellung eines transdermalen therapeutischen Systems, welches eine wirkstoffundurchlässige Rückschicht, ein Wirkstoffreservoir und eine ablösbare Schutzschicht aufweist, wobei das Wirkstoffreservoir haftklebend ist oder das TTS mindestens eine haftklebende Schicht aufweist.

## Claims

1. Transdermal therapeutic system (TTS) for administering at least one partial dopamine D2 agonist, having an active substance-impermeable backing layer, an active substance reservoir and a detachable backing layer, where the active substance reservoir is pressure-sensitive adhesive or the TTS has at least one pressure-sensitive adhesive layer, and where the active substance reservoir is configured as a matrix system or as a membrane system, **characterized in that** at least one of the partial dopamine D2 agonists contained in the active substance reservoir is aripiprazole.

2. TTS according to claim 1, **characterized in that** the active substance reservoir is configured as a single-, double- or multilayered active substance matrix.

3. TTS according to claim 2, **characterized in that** the active substance matrix is a plastics or synthetic resin matrix, preferably a pressure-sensitive adhesive matrix, where the basic polymer(s) of this matrix is/are preferably selected from the group comprising polymers based on acrylic acid and its esters, isobutylenes, ethylene-vinyl acetate copolymers, natural rubbers, synthetic rubbers such as styrene-diene copolymers, especially styrene-butadiene block copolymers, isoprene block polymers, acrylonitrile-butadiene rubber, butyl rubber and neoprene rubber, as well as pressure-sensitive adhesives based on silicone, as well as hot-melt adhesives, preferably mixtures of esters of hydrogenated colophony with cellulose derivatives.

4. TTS according to any one of the preceding claims, **characterized in that** the active substance reservoir contains a fibre material, a woven fabric or a nonwoven, to which the active substance is adsorbed.

5. TTS according to claim 1, **characterized in that** the active substance reservoir is configured as a pouch-shaped reservoir which contains the active substance in a flowable, viscous, semi-solid, gel-like or liquid preparation or solution and is confined on the side facing the skin by an active substance-permeable layer and on the side averted from the skin by an active substance-impermeable layer.

6. TTS according to any one of the preceding claims, **characterized in that** it additionally has an active substance-permeable membrane which modifies or controls the rate of active substance release.

7. TTS according to any one of the preceding claims, **characterized in that** aripiprazole is contained in a concentration in the range of from 0.1 to 50%-wt., preferably from 1 to 10%-wt, in each case relative to the total mass of the active substance reservoir.

8. TTS according to any one of the preceding claims, **characterized in that** aripiprazole is present in the active substance reservoir in dissolved state.

9. TTS according to any one of the preceding claims, **characterized in that** the active substance reservoir contains at least one solubilizer, preferably in an amount of from 1 to 50%-wt., with particular preference from 5 to 35%-wt., in each case relative to the total weight of the active substance reservoir.

10. TTS according to claim 9, **characterized in that** the solubilizers(s) is/are selected from the group comprising polyhydric alcohols, especially 1,2-propanediol, butanediol, glycerine, polyethylene glycol 400, tetrahydrofurfuryl alcohol, diethyleneglycol monoether, diethyltoluamide and monoisopropylidene glycerine.

11. TTS according to any one of the preceding claims, **characterized in that** the active substance reservoir contains at least one permeation-enhancing substance, preferably in an amount of from 0.1 to 25%-wt, with particular preference from 1 to 10%-wt, in each case relative to the total weight of the active substance reservoir.

12. TTS according to claim 8, **characterized in that** the permeation-enhancing substance(s) is/are selected from the group comprising fatty alcohols, preferably decanol and dodecanol, as well as fatty acids, preferably oleic acid, myristic acid, as well as polyoxyethylene fatty alcohol ethers, preferably polyoxylauryl ether, as well as polyoxyethylene fatty acid esters, fatty acid esters of sorbitane monolaurate, esters of long-chain fatty acids with methyl, ethyl or isopropyl alcohol, esters of fatty alcohols with acetic acid or lactic acid, as well as oleic acid diethanolamine.

13. TTS according to any one of the preceding claims, **characterized in that** it additionally contains at least one active substance selected from the group comprising phenothiazines and its analogues, butyrophenones and diphenylbutyl piperidines.

14. The use of aripiprazole for production of a transdermal therapeutic system comprising an active substance-impermeable backing layer, an active substance reservoir and a detachable protective layer, where the active substance reservoir is pressure-sensitive adhesive or the TTS has at least one pressure-sensitive adhesive layer.

## Revendications

1. Système thérapeutique transdermique (STT) pour l'administration d'au moins un agoniste partiel de la dopamine D2, qui présente une couche dorsale imperméable à la substance active, un réservoir de substance active et une couche protectrice détachable, dans lequel le réservoir de substance active adhère par contact, ou le STT présente au moins une couche adhérant par contact, et dans lequel le réservoir de substance active est formé en système à matrice ou en système à membrane, **caractérisé en ce qu'**au moins un des agonistes partiels de la dopamine D2 contenus dans le réservoir de substance active est l'aripiprazol.

2. STT selon la revendication 1, **caractérisé en ce que** le réservoir de substance active est formé en matrice de substance active à une, deux ou plusieurs couches.

3. STT selon la revendication 2, **caractérisé en ce que** la matrice de substance active est une matrice de matière synthétique ou de résine synthétique, de préférence une matrice adhérant par contact, le ou les polymère(s) de base de cette matrice étant de préférence choisi(s) dans le groupe qui comprend les polymères à base de l'acide acrylique et de ses esters, l'isobutylène, les copolymères éthylène-acétate de vinyle, les caoutchoucs naturels, les caoutchoucs synthétiques comme les copolymères styrène-diène, en particulier les copolymères séquencés styrène-butadiène, les copolymères séquencés d'isoprène, le caoutchouc acrylonitrile-butadiène, le caoutchouc butyle ou le caoutchouc néoprène, ainsi que les adhésifs par contact à base de silicone et les adhésifs par fusion à chaud, de préférence les mélanges d'esters de colophane hydrogénée avec des dérivés de cellulose.

4. STT selon l'une des revendications précédentes, **caractérisé en ce que** le réservoir de substance active contient un matériau fibreux, un tissu ou un non tissé, sur lequel la substance active est adsorbée.

5. STT selon la revendication 1, **caractérisé en ce que** le réservoir de substance active se présente sous forme de pochette réservoir qui contient la substance active dans une préparation ou solution fluide, visqueuse, semi-solide, géliforme ou liquide, et qui du côté de la peau est limitée par une couche perméable à la substance active, et, du côté éloigné de la peau, par une couche imperméable à la substance active.

6. STT selon l'une des revendications précédentes, **caractérisée en ce qu'**elle présente en outre une membrane perméable à la substance active, qui modifie ou règle la vitesse de libération de la substance active.

7. STT selon l'une des revendications précédentes, **caractérisé en ce qu'**il contient de l'aripiprazol en une concentration allant de 0,1 à 50 % en poids, de préférence de 1 à 10 % en poids, toujours par rapport à la masse totale du réservoir de substance active.

8. STT selon l'une des revendications précédentes, **caractérisé en ce que** l'aripiprazol se présente à l'état dissous dans le réservoir de substance active.

9. STT selon l'une des revendications précédentes, **caractérisé en ce que** le réservoir de substance active contient au moins un agent de solubilisation, de préférence en une quantité de 1 à 50 % en poids, de façon particulièrement préférée de 5 à 35 % en poids, toujours par rapport au poids total du réservoir de substance active.

10. STT selon la revendication 9, **caractérisé en ce que** le ou les agent(s) de solubilisation est ou sont choisi(s) dans le groupe qui comprend les alcools polyvalents, de préférence le 1,2-propanediol, les butanediols, la glycérine, le polyéthylèneglycol 400, l'alcool tétrahydrofurfurylique, le monoéther de diéthylèneglycol, le diéthyltoluamide et la monoisopropylidène-glycérine.

11. STT selon l'une des revendications précédentes, **caractérisé en ce que** le réservoir de substance active contient au moins une substance favorisant la perméation, de préférence en une quantité de 0,1 à 25 % en poids, de façon particulièrement préférée de 1 à 10 % en poids, toujours par rapport au poids total du réservoir de substance active.

12. STT selon la revendication 8, **caractérisé en ce que** la ou les substances favorisant la perméation est ou sont choisie(s) dans le groupe qui comprend les alcools gras, de préférence le décanol et le dodécanol, ainsi que les acides gras, de préférence l'acide oléique, l'acide myristique, ainsi que les éthers d'alcools gras de polyoxyéthylène, de préférence l'éther polyoxylaurique, ainsi que les esters d'acides gras de polyoxyéthylène, les esters d'acides gras de monolaurate de sorbitane, les esters d'acides gras à longue chaîne avec l'alcool méthylique, l'alcool éthylique ou l'alcool isopropylique, les esters d'alcools gras avec l'acide acétique ou l'acide lactique, ainsi que la diéthanolamine de l'acide oléique.

13. STT selon l'une des revendications précédentes, **caractérisé en ce qu'**il contient en outre au moins une substance active qui est choisie dans le groupe qui comprend les phénothiazines et leurs analogues, les butyrophénones et les diphénylbutylpipéridines.

14. Utilisation de l'aripiprazol pour la fabrication d'un système thérapeutique transdermique qui présente une couche dorsale imperméable à la substance active, un réservoir de substance active et une couche protectrice détachable, dans lequel le réservoir de substance active adhère par contact, ou le STT présente au moins une couche adhérant par contact.
